**Europäisches Patentamt**

**European Patent Office**

**Office européen des brevets**

(19)

(11) Veröffentlichungsnummer : **0 357 547 B1**

(12)
# EUROPÄISCHE PATENTSCHRIFT

(45) Veröffentlichungstag der Patentschrift :
**19.11.92 Patentblatt 92/47**

(51) Int. Cl.$^5$ : **A61F 2/36**

(21) Anmeldenummer : **89810530.9**

(22) Anmeldetag : **13.07.89**

(54) **Verankerungsschaft für eine Hüftgelenksprothese.**

(30) Priorität : **24.08.88 CH 3144/88**

(43) Veröffentlichungstag der Anmeldung :
**07.03.90 Patentblatt 90/10**

(45) Bekanntmachung des Hinweises auf die
Patenterteilung :
**19.11.92 Patentblatt 92/47**

(84) Benannte Vertragsstaaten :
**AT DE FR GB IT**

(56) Entgegenhaltungen :
**EP-A- 0 106 945**

(56) Entgegenhaltungen :
**EP-A- 0 169 976**
**EP-A- 0 182 176**
**DE-U- 8 236 213**
**FR-A- 2 602 672**

(73) Patentinhaber : **GEBRÜDER SULZER
AKTIENGESELLSCHAFT
Zürcherstrasse 9
CH-8401 Winterthur (CH)**

(72) Erfinder : **Willert, Hans-Georg Prof. Dr.-med.
Schlegelweg 9
W-3400 Göttingen (DE)**
Erfinder : **Koch, Rudolf
Oberdorfstrasse 229
CH-8267 Berlingen (CH)**

EP 0 357 547 B1

## Beschreibung

Die Erfindung betrifft einen Verankerungsschaft für eine Hüftgelenksprothese, der in seinem proximalen Bereich mit aus dem Schaftkörper hervorstehenden Vorsprüngen versehen ist, die in Richtung seiner Längsachse verlaufen und gegen diese in einem spitzen Winkel geneigt sind.

Ein Schaft der vorstehend genannten Art ist beispielsweise bekannt aus der EP-A-141 022. Bei dieser bekannten Konstruktion sind die Vorsprünge als sich über den ganzen proximalen Bereich erstreckende, durchgehende Rippen ausgebildet, bei denen der Winkel, den die Rippenkämme mit der Mittelebene des blattartigen Schaftes bilden grösser ist als derjenige der zwischen den Rippen liegenden Talsohlen. In der Praxis hat sich nun gezeigt, dass die durch diese Rippen bewirkende Verdrängung und Verdichtung des spongiosen Gewebes unter Umständen ungenügend ist. Darüberhinaus verursachen diese durchgehenden Rippen in gewissem Umfang eine Schneidwirkung bei ihrem Eindringen in die relativ weiche Spongiosa.

Konstruktiv sehr ähnlich ist der proximale Bereich des Verankerungsschaftes nach der FR-A-2 602 672 gestaltet.

Auch bei dieser bekannten Konstruktion haben die nach anterior und posterior gerichteten Blattseiten des Schaftes im proximalen Bereich als Vorsprünge durchgehende Rippen, deren Höhe von distal nach proximal zunimmt.

Aus der EP-A-0 182 176 schliesslich ist ein Verankerungsschaft bekannt, der in seinem proximalen Bereich, wie die vorstehend diskutierten Konstuktionen, nach proximal in der Höhe zunehmende, durchgehende Rippen aufweist; im distalen Bereich dieses Schaftes sind prismenartige Vorsprünge vorhanden, die auf der ganzen, von ihnen belegten Schaftoberfläche gleiche Form und eine konstante Grösse, insbesondere eine konstante Basisbreite haben und versetzt zueinander angeordnet sind, wobei die Versetzung einen Bruchteil der Basisbreite beträgt.

Aufgabe der Erfindung ist es, einen Verankerungsschaft zu schaffen, der verglichen mit den bekannten Konstruktionen eine erhöhte Verdrängungs- und Verdichtungswirkung für die Spongiosa und gleichzeitig eine verminderte Schneidwirkung hat, ohne dass die Gefahr einer Sprengwirkung unzulässig vergrössert wird, die das eindringende Implantat in Umfangsrichtung am Knochen hervorrufen kann.

Mit der Erfindung wird diese Aufgabe dadurch gelöst, dass die Vorsprünge - im proximalen Bereich - als einzelne pyramidenartige Prismen mit nach distal gerichteten Spitzen ausgebildet sind, dass ferner die aus dem Schaftkörper hervorstehenden Basishöhen der Prismen bei proximal gelegenen Prismen grösser sind als bei distal gelegenen, und dass schliesslich das Verhältnis der Basishöhe eines Prismas zu seiner Sehne in Richtung der Schaftlängsachse bei den Prismen konstant ist.

Die Ausbildung der Vorsprünge als einzelne Prismen ermöglicht, die Prismenbasen relativ breit auszuführen und so eine verstärkte Verdichtung der Spongiosa zu erreichen; die aus dem Schaftkörper hervorstehenden grösseren Basishöhen der proximal gelegenen Prismen relativ zu denjenigen der weiter distal gelegenen bewirken am Schaftblatt gesamthaft eine Keilwirkung; ein konstantes Verhältnis der Basishöhe eines Prismas zu seiner Sehne in Richtung der Schaftlängsachse ergibt eine relativ homogene Verteilung der Winkel für die resultierenden, von den Flanken der Prismen auf den Knochen ausgeübten Kräfte. Beide Massnahmen verringern die Sprengwirkung, mit der das eindringende Implantat den Knochen in Umfangsrichtung belastet.

Wenn die Basisbreiten der Prismen konstant gehalten werden, ist die Verdrängung oder Verdichtung der Spongiosa ebenfalls weitgehend konstant. Werden mindestens einzelne Prismen in Richtung senkrecht zur Schaftlängsachse versetzt zueinander angeordnet, so fluchten ihre Achsen beim Einschlagen des Schaftes nicht miteinander, wodurch die Schneidwirkung der in die Spongiosa eindringenden Prismen herabgesetzt wird.

Andererseits kann es jedoch zweckmässig sein, die aus dem Schaftkörper hervorragenden Prismen scharfkantig auszubilden, um ihr Eindringen in die Spongiosa zu erleichtern. Im folgenden wird die Erfindung anhand eines Ausführungsbeispiels im Zusammenhang mit der Zeichnung näher erläutert.

Fig. 1 zeigt eine Ansicht in Richtung ventral oder dorsal von dem proximalen Teil eines Verankerungsschaftes für eine Femurkopfprothese;

Fig. 2 ist eine Aufsicht auf Fig. 1 von oben, während

Fig. 3 eine Ansicht der Fig. 1 von links darstellt.

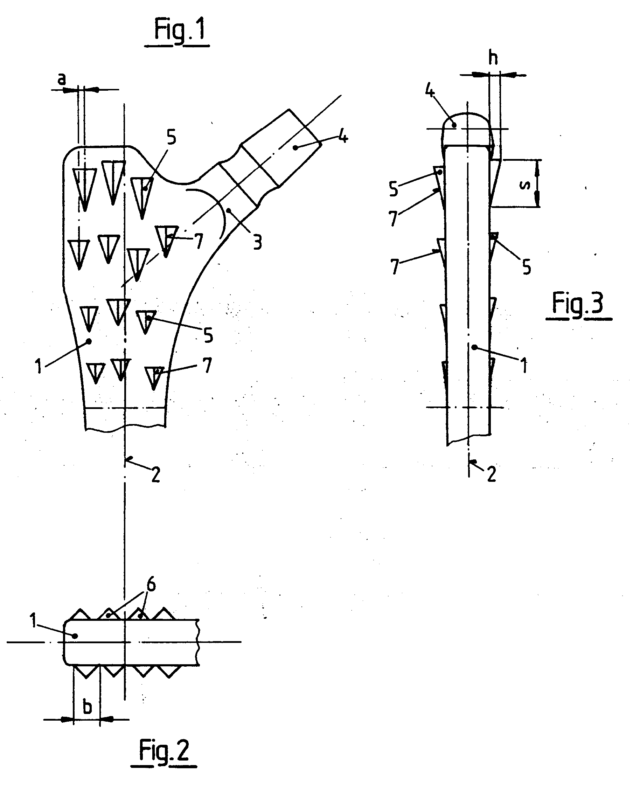

Der nur in seinem proximalen Bereich gezeigte Verankerungsschaft 1 ist ein blattartiger Geradschaft mit rechteckigem Querschnitt (Fig. 2); seine Längsachse ist mit 2 bezeichnet. Der in Fig. 1 nach rechts oben weisende Prothesenhals 3 ist kragenlos an den Schaft 1 angesetzt und trägt einen konischen Zapfen 4 für die Aufnahme eines nicht dargestellten Gelenkkopfes.

Die nach anterior oder posterior weisenden Blattseiten tragen über den proximalen Bereich verteilt als Verankerungsstruktur prismenartige Vorsprünge 5, deren Querschnittsflächen als gleichschenklige Dreiecke ausgebildet sind. Die Basen 6 (Fig. 2) der Prismen 5 sind nach proximal gerichtet und haben eine konstante Breite b; die Kanten 7 der Prismen 5 verlaufen parallel zur Längsachse 2 des Schaftes 1 und sind scharfkantig ausgeführt.

Die Höhe der Kante 7 in einer Basis 6 ist mit h bezeichnet, während die Prismensehne, d.h. die Projektion der Prismenkante 7 in die Ebene des Schaftblattes, mit s benannt ist.

Aus Fig. 3 ist zu entnehmen, dass die Basishöhen h der Prismen 5 von proximal nach distal geringer werden, wodurch sich relativ zum Schaftblatt gesamthaft eine Keilwirkung ergibt, mit der die Verdichtung der Spongiosa gefördert wird.

Weiterhin sind die einzelnen Prismen 5 sowohl in Richtung der Schaftlängsachse 2 als auch in Richtung senkrecht dazu auf der Oberfläche des Schaftblattes gegeneinander versetzt, um - wie erwähnt - die Schneidwirkung der Struktur abzuschwächen. Senkrecht zur Schaftlängsachse beträgt die Versetzung a dabei mit Vorteil 1/4 bis 3/4 der Basisbreite b.

Neben der Basisbreite b ist auch das Verhältnis der Basishöhe h zur Sehne s jedes Prismas 5 konstant gehalten, wodurch - in Verbindung mit der Keilwirkung, die durch die von distal nach proximal stetig grösser werdenden Basishöhen h der Prismen 5 gesamthaft erreicht wird, -vom eindringenden Implantat und seiner Verankerungsstruktur auf den Knochen in Umfangsrichtung ausgeübte Sprengkräfte verringert werden.

## Patentansprüche

1. Verankerungsschaft (1) für eine Hüftgelenksprothese, der in seinem proximalen Bereich mit aus dem Schaftkörper hervorstehenden Vorsprüngen (5) versehen ist, die in Richtung seiner Längsachse (2) verlaufen und gegen diese in einem spitzen Winkel geneigt sind, **dadurch gekennzeichnet**, dass die Vorsprünge als einzelne pyramidenartige Prismen (5) mit nach distal gerichteten Spitzen ausgebildet sind, dass ferner die aus dem Schaftkörper (1) hervorstehenden Basishöhen (h) der Prismen (5) bei proximal gelegenen Prismen (5) grösser sind als bei distal gelegenen, und dass schliesslich das Verhältnis (h/s) der Basishöhe (h) eines Prismas (5) zu seiner Sehne (s) in Richtung der Schaftlängsachse (2) bei den Prismen (5) konstant ist.

2. Verankerungsschaft nach Anspruch 1, dadurch gekennzeichnet, dass die aus dem Schaftkörper (1) hervorragenden Kanten (7) der Prismen (5) scharfkantig ausgeführt sind.

## Claims

1. An anchoring stem (1) for a hip joint prosthesis, the stem being provided in its proximal zone with projections (5) which project from the body of the stem, run parallel to its longitudinal axis (2) and are inclined at an acute angle to this axis, characterised in that the projections are in the form of individual pyramid-like prisms (5) pointing in the distal direction, in addition the prism base heights (h) projecting from the body (1) of the stem are greater in proximal prisms (5) than in distal prisms, and finally the ratio (h/s) of the base height (h) of a prism (5) to its chord (s) parallel to the longitudinal axis (2) of the stem is constant in the prisms (5).

2. An anchoring stem as claimed in claim 1, characterised in that the prism edges (7), which project from the body (1) of the stem, are sharp-edged.

## Revendications

1. Tige (1) d'ancrage d'une prothèse d'articulation de la hanche, dont la région proximale comporte des protubérances (5) qui sont saillantes sur le corps de la tige, qui sont orientées dans la direction de son axe longitudinal (2) et qui sont inclinées sur ce dernier en inscrivant avec lui un angle aigu, caractérisée en ce que les protubérances sont conformées en prismes individuels pyramidaux (5) dont la pointe est dirigée vers le côté distal, en ce que par ailleurs la hauteur (h) de la base, qui est saillante sur le corps (1) de la tige, des prismes (5) situés du côté proximal est supérieure à celle des prismes situés du côté distal et en ce que finalement le rapport (h/s) de la hauteur (h) de la base d'un prisme (5) à sa corde (s) orientée dans la direction de l'axe longitudinal (2) de la tige est constant pour tous les prismes (5).

2. Tige d'ancrage selon la revendication 1, caractérisée en ce que les arêtes (7) des prismes (5) qui sont saillantes sur le corps (1) de la tige sont vives.

## Fig.1

## Fig.3

## Fig.2